# EUROPÄISCHE PATENTSCHRIFT

(11) **EP 2 595 578 B1**
(45) Veröffentlichungstag und Bekanntmachung des Hinweises auf die Patenterteilung: **19.07.2017**
(21) Anmeldenummer: 11735803.6
(22) Anmeldetag: 21.07.2011
(51) Int. Cl.: A61F 2/64, A61F 2/74, A61F 2/50, A61F 2/38, A61F 2/68, A61F 2/80, F04B 33/00

(54) **PROTHESENKNIEGELENK**
KNEE JOINT PROSTHESIS
PROTHESE DE L'ARTICULATION DU GENOU

(30) Priorität: 21.07.2010 DE 102010031723
(43) Veröffentlichungstag der Anmeldung: 29.05.2013
(73) Patentinhaber: Otto Bock HealthCare GmbH, 37115 Duderstadt (DE)
(72) Erfinder: BOITEN, Herman, 37085 Göttingen (DE)
(74) Vertreter: Stornebel, Kai
(86) Internationale Anmeldenummer: PCT/EP2011/003658
(87) Internationale Veröffentlichungsnummer: WO 2012/010309

(56) Entgegenhaltungen:
- DE-A1- 4 233 247
- DE-U1- 29 905 842
- DE-U1-202009 012 627
- US-A1- 2006 212 130

## Beschreibung

Die Erfindung betrifft ein Prothesenkniegelenk mit einem Oberteil, das obere Anschlussmittel aufweist, und einem schwenkbar an dem Oberteil gelagerten Unterteil, das untere Anschlussmittel aufweist. Die oberen und unteren Anschlussmittel dienen zur Befestigung des Prothesenkniegelenkes an sich distal und proximal anschließende Komponenten. Die unteren Anschlussmittel dienen in der Regel zur Befestigung an einem Unterschenkelschaft, an dem wiederum ein Prothesefuß befestigt ist. Die oberen Anschlussmittel dienen oder das obere Anschlussmittel dient in der Regel zur Befestigung eines Oberschenkelschaftes, der zur Aufnahme eines Oberschenkelstumpfes dient.

Um einem Prothesenträger eine ausreichende Sicherheit während des Tragens der Prothese zu geben, ist es notwendig, dass die Prothese sicher und fest an dem Stumpf angeordnet ist. Befestigungen über Gurte, Schlaufen und Schnallen haben den Nachteil, dass der Stumpf eingeschnürt werden kann und dass während des Gehens Volumenschwankungen des Stumpfes nicht berücksichtigt werden können. Es hat sich daher die Anwendung von so genannten Saugschäften bewehrt, die in der Regel aus einem weichen, luftdichten Innenschaft bestehen, der an einem formstabilen Außenschaft angeordnet oder festgelegt ist. An dem Außenschaft befinden sich Anschlusseinrichtungen zur Befestigung des Prothesenkniegelenkes, also Aufnahmen für ein oberes Anschlussmittel. Der Innenschaft oder Liner liegt vollflächig an dem Oberschenkelstumpf an und schließt im Wesentlichen luftdicht mit dem Außenschaft ab. Der Zwischenraum zwischen dem Liner und dem Außenschaft wird evakuiert, so dass über den Unterdruck eine Fixierung des Liners an dem Außenschaft erreicht wird. Über die Haftkraft des Liners an dem Oberschenkelstumpf wird somit eine stabile Kopplung zwischen dem Prothesenkniegelenk und dem Stumpf zu erreichen.

Die DE 10 2004 036 669 A1 beschreibt eine Pumpe mit wenigstens einer flexiblen Wandlung eines abgeschlossenen Fluidvolumens, die mittels einer ersten Kraft in Richtung Volumenverkleinerung und mittels einer zweiten Kraft nach einer vorhergehenden Volumenverkleinerung in Richtung Volumenvergrößerung verformbar ist. Die Pumpe weist ein Einlassventil mit
einer Einlassleitung und ein Auslassventil mit einer Auslassleitung des Fluidvolumens auf. An der flexiblen Wandlung ist ein elastisches Material flächig angelegt, das bei einer Verformung der flexiblen Wandlung durch eine der Kräfte zusammengepresst wird und dessen Rückstellkraft nach Beendigung der Krafteinwirkung die Wandlung zurückverlagert. Die Pumpe kann als Vakuumpumpe verwendet werden, die beim Auftreten aufgrund des Körpergewichts ein Vakuum erzeugt. Als Einsatzgebiet wird eine Vakuumunterstützung eines Saugschaftes angegeben. Die DE 601 26 154 T2 beschreibt eine Vakuumpumpe in einem Stoßdämpfer, die gewichtsbasiert aktiviert wird.

Die DE 299 05 842 U1, die als nächstliegender Stand der Technik angesehen wird, beschreibt ein künstliches Kniegelenk mit einem Schwungphasen-Steuerelement, das für unterschiedliche schnelle Bewegungsabläufe geeignet ist. In einem Zylinder des künstlichen Kniegelenks ist ein Kolbenelement angeordnet, das den Zylinder in eine obere und eine untere Kammer teilt. Die obere Kammer weist in dem Zylinder eine Bohrung auf, die untere hingegen zwei Bohrungen. Eine Verbindungskonstruktion ist mit dem Kolben verbunden, um diesen zu bewegen, wenn das Kniegelenk betätigt wird. Das Schwungphasen-Steuerelement ist mit den Durchgangslöchern in dem Zylinder verbunden und weist ein Hebelelement auf, das als Reaktion auf einem Luftdruck eine Drosselventil-Einheit verstellt.

Die DE 42 33 247 A1 beschreibt eine Schwungphasensteuervorrichtung für ein künstliches Prothesenkniegelenk mit einer Kolben-Zylindereinrichtung. Der Kolben trennt zwei Kammern voneinander, die durch eine erste Drossel miteinander verbindbar sind. Auf der Ausgangsseite der zweiten Kammer ist eine zweite Drossel vorgesehen, die die zweite Kammer mit einem Fluidreservoir verbindet. Ein Steuerkolben wird von dem Druck in der zweiten Kammer dergestalt beaufschlagt, dass er die zweite Drossel schließt, wenn der Druck in der zweiten Kammer einen vorbestimmten Wert überschreitet. Wenn die zweite Drossel geschlossen ist, unterstützt der elastische Druck in der zweiten Kammer die Umkehrbewegung des Kolbens in dem Zylinder, so dass eine Extensionsunterstützung erfolgt.

Die US 2006/0212130 A1 betrifft eine prothetische Vorrichtung zur Befestigung an einem Stumpf. Ein Prothesenknöchelgelenk ist über ein Fangband mit einem Kolben in dem Zylinder einer Pumpe verbunden. Über die Pumpe wird Luft aus einem Prothesenschaft abgezogen, um diesen sicher an einem Stumpf zu halten.

Gewichtsbasierte Vakuumpumpen weisen häufig einen geringen Verlagerungsweg auf, da eine Axialverlagerung während des Gehens oder Stehens nur in einem begrenzten Maße gewünscht oder zulässig ist, um ein Absacken des Körpers während des Gehens zu vermeiden. Eine Kopplung der Vakuumpumpe mit einem Stoßdämpfer kann zu Schwierigkeiten bei der Schwungphasensteuerung führen.

Aufgabe der vorliegenden Erfindung ist es, ein Prothesenkniegelenk bereitzustellen, das verbesserte Mittel zur Festlegung an einem Oberschenkelstumpf aufweist.

Erfindungsgemäß wird diese Aufgabe durch ein Prothesenkniegelenk mit den Merkmalen des Hauptanspruches gelöst. Vorteilhafte Ausgestaltungen und Weiterbildungen der Erfindung sind in den Unteransprüchen, der Beschreibung und den Figuren aufgeführt.

Das erfindungsgemäße Prothesenkniegelenk mit einem Oberteil, das obere Anschlussmittel aufweist, und einen schwenkbar an dem Oberteil gelagerten Unterteil, das untere Anschlussmittel aufweist, sieht vor, dass eine durch die Relativbewegung des Oberteils und dem Unterteil angetriebene Vakuumpumpe mit einem Eingang und einem Ausgang dem Prothesenkniegelenk zugeordnet ist und die Vakuumpumpe in dem Oberteil integriert und mit einem Saugschaft verbunden ist. Durch die Übertragung der Relativbewegung des Oberteils zu dem Unterteil, also eine rotatorischen Bewegung, auf die Vakuumpumpe ist es möglich, einen relativ langen Verstellweg bereitzustellen, so dass ein großes Volumen in der Vakuumpumpe bewegt werden kann. Darüber hinaus wirken in dem Kniegelenk während der Bewegung große Kräfte, die leicht zur Erzeugung des Vakuums verwendet werden können. Dadurch ist es auch möglich, eine große Übersetzung der Relativbewegung zwischen dem Oberteil und dem Unterteil über ein Getriebe zu nutzen, so dass auch bei geringen Flexions- oder Extensionswinkeln eine große Verlagerung und ein großer Verstellweg der Vakuumpumpe erreicht werden können. Ein Rückschlagventil ist vorgesehen, das ein Rückströmen von Luft in eine Saugleitung oder in einen Saugraum verhindert, so dass aus dem Zwischenraum zwischen Liner und Außenschaft abgepumpte Luft nicht in den Zwischenraum rückströmen kann. Dadurch wird der Unterdruck aufrechterhalten. Eine Auslassleitung kann ebenfalls mit einem Rückschlagventil versehen sein, so dass die Vakuumpumpe stets Luft aus dem dafür vorgesehenen Raum zwischen Liner und Außenschaft absaugen kann. Gemäß der Erfindung ist die Vakuumpumpe direkt in dem Oberteil integriert, so dass die in der Regel massiven Komponenten des Oberteils und des Unterteils zusätzlich genutzt werden, indem eine relativ kompakte Struktur in Gestalt der Vakuumpumpe eingebaut wird. Dadurch ist es möglich, ohne Verringerung der strukturellen Festigkeit des Oberteils oder des Unterteils ein weiteres Funktionselement zu integrieren. Sofern die Vakuumpumpe in dem Oberteil integriert ist, ergibt sich der Vorteil, dass eine direkte Verbindung zwischen dem Außenschaft und der Vakuumpumpe hergestellt werden kann, ohne dass eine komplexe Schlauchführung vorgesehen sein muss. Die Verbindung zwischen dem zu evakuierenden Zwischenraum und der Vakuumpumpe kann starr ausgebildet sein, da keine Relativbewegung zwischen dem Außenschaft und der Vakuumpumpe auftritt.

Die Vakuumpumpe kann so ausgebildet sein, dass während der Flexion ein Unterdruck erzeugt wird, während der Extension ein Ausstoß aus der Pumpenkammer erfolgt. Während der Flexion wirken in der Regel größere Kräfte, so dass die Flexionsbewegung besser geeignet ist, ein Vakuum zu erzeugen als die Extensionsbewegung.

Vorteilhafterweise ist in der Vakuumpumpe ein Saugkolben angeordnet, der aufgrund der Relativbewegung zwischen dem Oberteil und dem Unterteil innerhalb des Pumpenzylinders oder in dem Kolbenraum verlagert wird. Der Saugkolben kann dabei als Schwenkkolben oder Linearkolben ausgebildet sein. Bei einer Ausgestaltung als Linearkolben erfolgt eine Umwandlung der Rotationsbewegung in eine Linearbewegung, bei der Anwendung eines Schwenkkolbens kann die Rotationsbewegung des Oberteils zum Unterteil beibehalten werden. Zwischen dem Oberteil und dem Unterteil ist vorzugsweise eine Kraftübertragungseinrichtung angeordnet, um die Relativbewegung des Unterteils relativ zum Oberteil auf die Vakuumpumpe zu übertragen. Gegebenenfalls kann eine Übersetzung durch ein Zahnradgetriebe oder durch ein Hebelgetriebe erfolgen, so dass eine Kraft- oder Wegvergrößerung erreicht werden kann. Ebenfalls ist es möglich, dass ein Zahnrad oder mehrere Zahnräder zur Kraftübertragung vorgesehen sind, so dass ein Zahnradgetriebe oder ein Zahnrad-Hebelgetriebe eingesetzt werden kann, um die Pumpe anzutreiben. Das Prothesenkniegelenk kann als monozentrisches oder als polyzentrisches Kniegelenk ausgebildet sein. In beiden Ausgestaltungen kann eine Vakuumpumpe verwendet werden, die durch die Relativbewegung des Oberteils relativ zum Unterteil angetrieben wird.

Um Ausstoßgeräusche zu dämpfen, ist vor dem Auslass oder in dem Auslass ein Dämpfer angeordnet.

Die Vakuumpumpe kann jedoch in jeder beliebigen Anordnung und Ausgestaltung ausgeführt werden, solange sie durch die Relativbewegung des Oberteils zu dem Unterteil angetrieben wird. Sie kann insbesondere selbst das Gelenk bilden. Sie kann insbesondere auch mit einem Teil der Kraftübertragungseinrichtung verbunden sein oder einen Teil dieser Kraftübertragungseinrichtung bilden.

Nachfolgend werden Ausführungsbeispiele der Erfindung anhand der Figuren näher erläutert. Es zeigen:
- Figur 1: eine Frontalansicht eines Prothesenkniegelenkes;
- Figur 2a: eine Schnittansicht eines Oberteils in Frontalebene;
- Figur 2b: eine Variante der Figur 2a während der Evakuierung;
- Figur 2c: eine Variante der Figur 2a während des Ausstoßens;
- Figur 3: eine schematische Darstellung der Konstruktion;
- Figur 4: eine schematische Darstellung des Funktionsprinzips während des Betriebs;
- Figur 5: eine Schnittdarstellung des Oberteils in Sagittalebene;
- Figur 6: eine Variante der Figur 5 in geschlossener Stellung;
- Figur 7a: ein Prothesenkniegelenk gemäß Figur 1 in Seitenansicht in Extensionsstellung,
- Figur 7b: ein Prothesenkniegelenk gemäß Figur 1 in Seitenansicht in Flexionsstellung
- Figur 8: eine Variante eines Prothesenkniegelenkes in Seitenansicht;
- Figur 9: eine Schnittansicht der Figur 8 in Sagittalebene;
- Figur 10: eine Detailansicht in Schnittdarstellung;
- Figur 11: eine Detailansicht in Rückansicht.

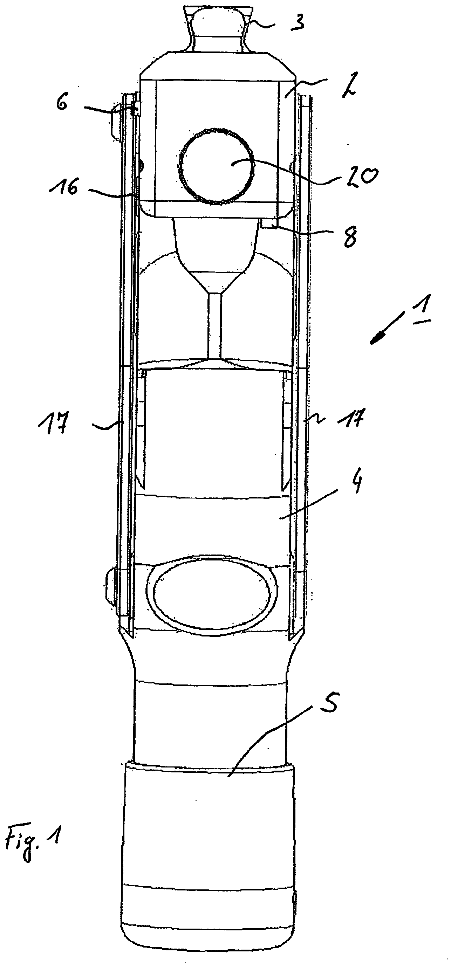

In der Figur 1 ist in Frontalansicht ein Prothesenkniegelenk 1 mit einem Oberteil 2 gezeigt, das obere Anschlussmittel 3 in Gestalt eines Befestigungspylons aufweist. Die oberen Anschlussmittel 3 dienen zur Befestigung des Oberteils 2 und damit des gesamten Prothesenkniegelenkes 1 an einem nicht dargestellten Oberschenkelschaft, über den das Prothesenkniegelenk 1 an dem Körper des Prothesennutzers festgelegt wird. Der Oberschenkelschaft dient in der Regel zur Aufnahme des Oberschenkelstumpfes, andere Befestigungsmöglichkeiten können ebenfalls vorgesehen sein. In der Regel sieht der Oberschenkelschaft eine im Wesentlichen geschlossene, oben offene Hülse vor, in die der Stumpf eingeführt wird. Vor dem Einführen des Stumpfes wird ein sogenannter Liner, in der Regel ein Silikonliner, über den Stumpf gezogen. Anschließend wird der Liner in den Außenschaft eingeführt und am oberen Rand des Außenschaftes umgeschlagen, so dass eine im Wesentlichen luftdichte Kammer zwischen der Außenseite des Liners und der Innenseite des Außenschaftes entsteht. Durch Anlegen eines Unterdruckes ist es möglich, die Fixierung des Liners und damit des Oberschenkelstumpfes in dem Außenschaft zu bewirken oder zu verbessern.

Das Oberteil 2 ist relativ zu einem Unterteil 4 gelenkig gelagert. An dem Unterteil 4 sind untere Anschlussmittel 5 zur Befestigung beispielsweise eines Unterschenkelschaftes und eines Prothesenfußes vorgesehen. Ebenfalls können in dem Unterteil 4 Dämpfungseinrichtungen, Antriebe und/oder Steuerungseinrichtungen vorgesehen sein, um die Relativbewegung zwischen dem Oberteil 2 und Unterteil 4 zu beeinflussen. Das Unterteil 4 kann über eine einzige Schwenkachse 16 relativ zu dem Unterteil 4 verschwenkbar gelagert sein, alternativ kann bei einem polyzentrischen Kniegelenk eine zusammengesetzte Verschwenkbewegung mit wandernden Momentanpolen gebildet werden, so dass die Schwenkbewegung des Oberteils 2 relativ zu dem Unterteil 4 nicht um eine fest an dem Unterteil 4 angeordnete Schwenkachse, sondern durch eine ortsveränderliche Momentanschwenkachse definiert ist.

Seitlich neben dem Oberteil 2 und dem Unterteil 4 sind Hebel 17 angeordnet, deren Funktion später näher erläutert wird.

An dem Oberteil 2 sind ein seitlicher Saugluftanschluss 6 und ein nach unten orientierter Auslass 8 vorgesehen. Der Saugluftanschluss 6 dient zum Anschluss einer Vakuumpumpe 20, die in dem dargestellten Ausführungsbeispiel in dem Oberteil 2 integriert ist, an einen Saugschaft, der Auslass 8 dient bei einer rückkehrenden Bewegung eines Saugkolbens dazu, Druckluft aus dem Zylinder der Vakuumpumpe 20 austreten zu lassen.

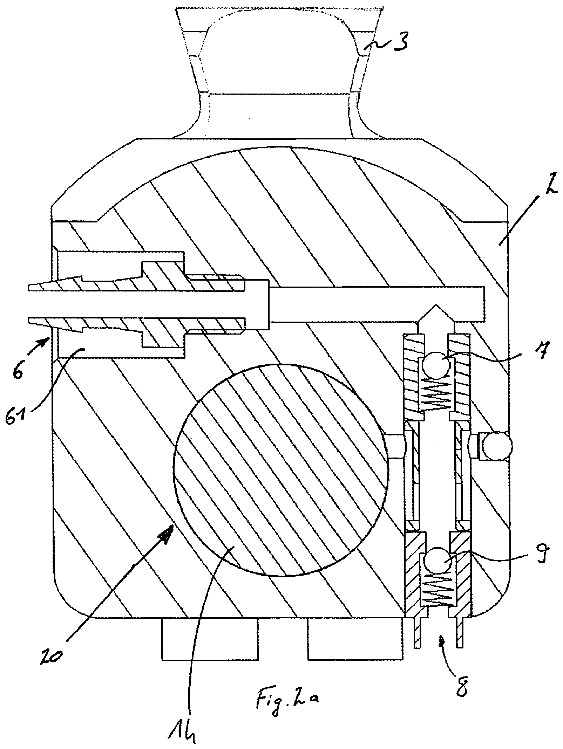

In der Figur 2a ist das Oberteil 2 in einer Schnittdarstellung gezeigt. Der Schnitt verläuft durch die Frontalebene des Oberteils 2. Ein oberes Anschlussmittel 3 in Gestalt eines Befestigungspylons ist entweder an dem Oberteil 2 angeschraubt oder einstückig damit ausgebildet. In dem Oberteil 2 sind weiterhin der Saugluftanschluss 6 und der Auslass 8 eingeführt. Der Saugluftanschluss 6 ist in eine Bohrung 61 eingeschraubt. Die Bohrung 61 dient als Kanal zur Weiterleitung der aus dem nicht dargestellten Oberschenkelschaft abgesaugten Luft zu der Vakuumpumpe 20 über eine Stichleitung 68. Von der Vakuumpumpe 20 ist der Saugkolben 14 zu sehen, der eine Reziprokbewegung innerhalb der Oberteils 2 ausführt um während der Flexion oder Extension eine hin- und hergehende Bewegung auszuführen. Von der Bohrung 61 wird die Saugluft durch einen Rückschlagventil 7 zu dem Saugkolben 14 geleitet. Das Rückschlagventil 7 verhindert bei einer rückkehrenden Bewegung des Saugkolbens 14 ein Zurückströmen der Druckluft in den Saugschaft. Um die Rückwärtsbewegung des Saugkolbens 14 nicht zu blockieren, ist ein Rückschlagventil 9 vor dem Auslass 8 vorgesehen, durch das die Druckluft ausströmen kann.

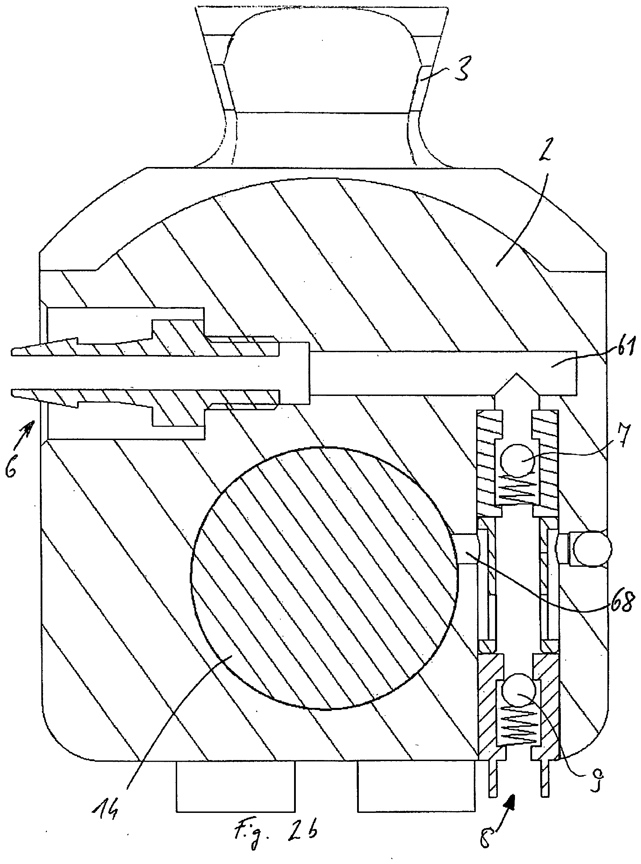

In der Figur 2b ist die Ventilstellung während des Ansaugens von Luft dargestellt, das obere Rückschlagventil 7 ist geöffnet, so dass von dem Saugluftanschluss 6 durch die die Bohrung 61 Luft in den Saugraum einströmen kann. Das Rückschlagventil 9 verhindert ein Einströmen von Umgebungsluft durch den Auslass 8.

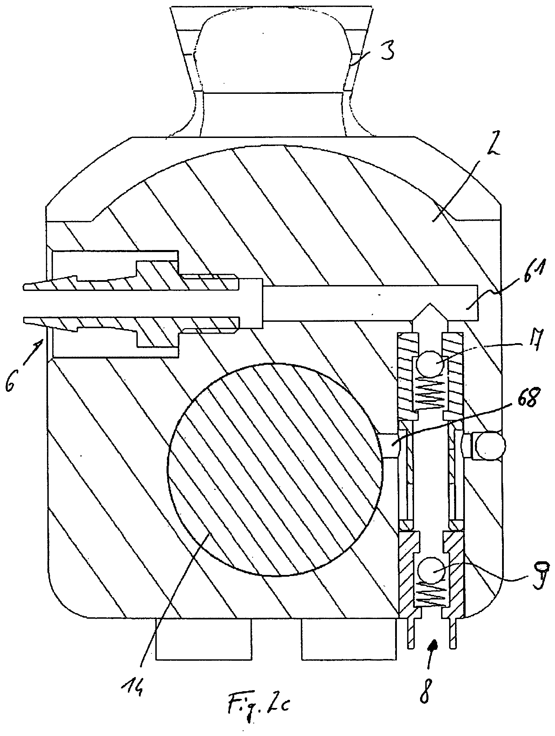

In der Figur 2c ist das dem Auslass 8 zugeordnete Rückschlagventil 9 geöffnet, durch den Saugkolben 14 komprimierte Luft kann in die Umgebung ausströmen.

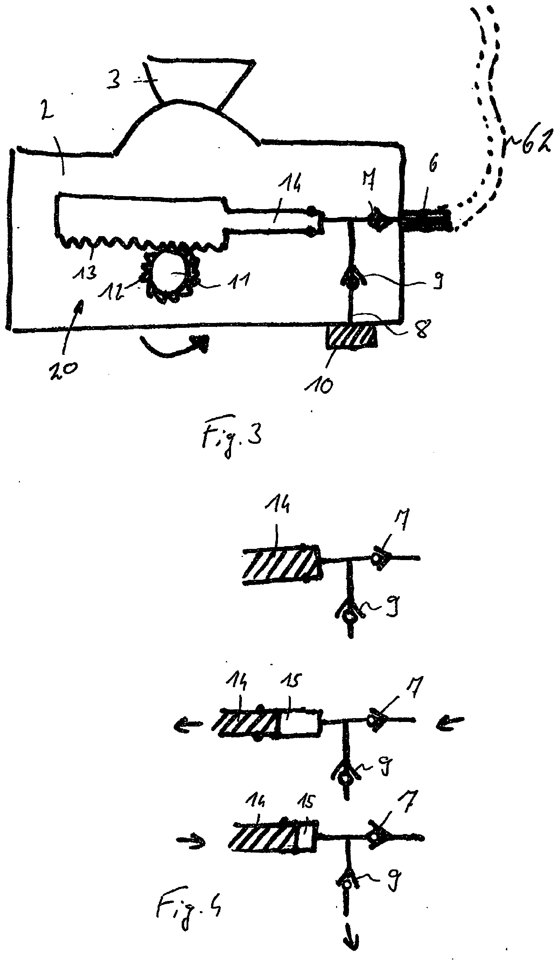

Der schematische Aufbau der Pumpe 20 ist in der Figur 3 dargestellt. An dem Oberteil 2 ist an dem Saugluftanschluss 6 eine Saugleitung 62 angeordnet, die zu einem Saugluftanschluss an einem nicht dargestellten Oberschenkelschaft führt. Ebenfalls dargestellt sind die Rückschlagventile 7 und 9 zur Durchflussbegrenzung in der jeweiligen Strömungsrichtung. An dem Ausgang des Auslasses 8 ist ein Schalldämpfer 10 zur Geräuschdämpfung der ausgeschobenen Luft angeordnet. Der Dämpfer 10 kann ebenfalls in dem Auslass 8 angeordnet sein. Innerhalb des Oberteils 2 ist die Vakuumpumpe 20 angeordnet, die einen Saugkolben 14 aufweist, der mit einer Zahnstange 13 verbunden ist. Die Zahnstange 13 kämmt ein Zahnrad 12, das an einer Welle 11 gelagert ist. Bei einer Flexionsbewegung des Prothesenkniegelenkes und einer Verlagerung des Oberteils 2 relativ zu dem Unterteil 4 wird bei einer Relativbewegung der Zahnstange 13 zu dem Zahnrad 12 eine Verlagerung des Saugkolbens 14 bewirkt, wodurch ein Unterdruck erzeugt wird, so dass Luft durch die Saugleitung 62 und den Saugluftanschluss 6 in den durch den Saugkolben 14 freigegebenen Raum angesaugt wird. Die Flexionsbewegung, die durch den Pfeil angedeutet wird, führt zu einer Verlagerung des Saugkolbens 14 in der Figur 3 nach links. Die Relativbewegung kann dadurch bewirkt werden, dass die Welle 11 rotatorisch ortsfest bleibt, während das Oberteil 2 sich um die Welle 11 herumdreht.

In der Figur 4 sind drei Stellungen des Saugkolbens 14 dargestellt, die mit der Stellung des Prothesenkniegelenkes korrelieren. In der oberen Darstellung ist der Saugkolben 14 vollständig eingeschoben. Das Prothesenkniegelenk befindet sich in der gestreckten Position. Beide Rückschlagventile 7, 9 sind geschlossen.

In der mittleren Darstellung ist gezeigt, dass sich das Prothesenkniegelenk in einer Flexionsbewegung befindet, so dass sich der Saugkolben 14 aus dem Zylinder 15 der Pumpe 20 herausbewegt. Dadurch wird ein Saugvolumen freigegeben, so dass Luft aus dem Schaft durch das erste Rückschlagventil 7 in den Zylinder 15 einströmen kann. Wird eine Extensionsbewegung eingeleitet, so dass sich das Unterteil 4 in Richtung nach vorne bewegt, verlagert sich auch der Saugkolben 14 in Pfeilrichtung und verringert das Saugvolumen, die dadurch komprimierte Luft innerhalb des Zylinders 15 weicht durch das Rückschlagventil 9, 10 aus dem Auslass 8 hinaus.

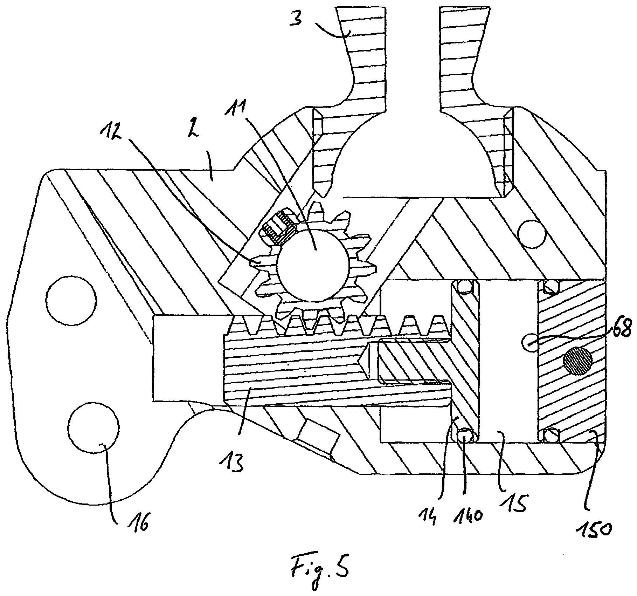

In der Figur 5 ist in einer Schnittdarstellung in Sagittalebene ein Oberteil 2 mit einem eingeschraubten oberen Anschlussmittel 3 in Gestalt eines Adapters gezeigt. Unterhalb des Adapters ist auf einer Welle 11 ein Zahnrad 12 montiert. Die Welle 11 ist schwenkbar innerhalb des Oberteils 2 angeordnet und wird über die Hebel 17, die in der Figur 1 gezeigt sind, bei einer Verschwenkbewegung des Oberteils 2 um eine Schwenkachse 16 relativ zu dem Oberteil 2 verdreht. Dies wird dadurch erreicht, dass die Welle 11 drehfest an den Hebeln 17 gelagert ist.

Weiterhin ist innerhalb des Oberteils 2 eine Zahnstange 13 angeordnet, die mit dem Zahnrad 12 kämmt. Der Zahnstange 13 ist ein Saugkolben 140 zugeordnet, im dargestellten Ausführungsbeispiel ist der Saugkolben 14 in die Zahnstange 13 eingeschraubt. Der Saugkolben 14 ist über eine Dichtung 14 gegenüber dem Zylinder 15 abgedichtet. Ein Verschlussstopfen 150 bildet den Abschluss des Saugraumes auf der dem Saugkolben 14 gegenüberliegenden Seite. Der Stichkanal 68 zu der nicht dargestellten Ventilanordnung mündet in den Saugraum. Der Saugkolben 14 ist von dem Verschlussstopfen 150 beabstandet dargestellt, das heißt, dass in dem Zylinder 15 durch die Bewegung des Saugkolbens 14 von dem Verschlussstopfen 150 weg ein Vakuum gebildet wurde, wodurch Luft aus dem Saugschaft durch den Saugluftanschluss 6, die Bohrung 61 und den Stichkanal 68 angesaugt wurde. Die Zahnstange 13 und der Saugkolben 14 befinden sich noch nicht in maximal entfernter Stellung von dem Verschlussstopfen 150, was wiederum bedeutet, dass keine maximale Flexion des Prothesenkniegelenkes und eine maximale Verlagerung des Oberteils relativ zu dem Unterteil erfolgt ist.

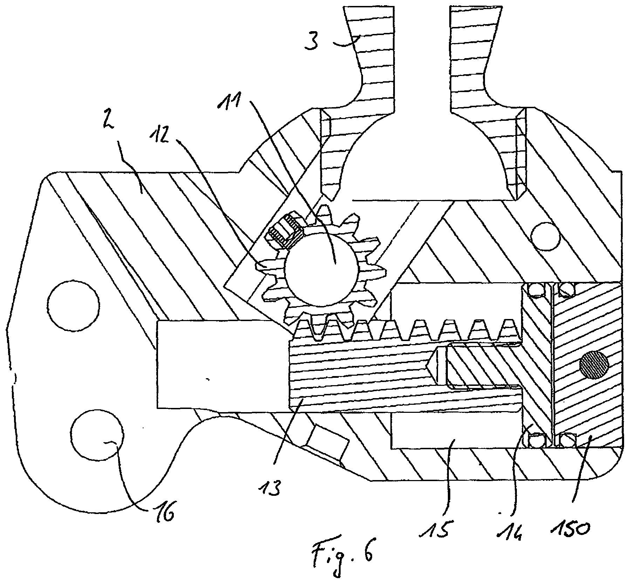

In der Figur 6 ist die Variante gemäß Figur 5 in einer maximal gestreckten Stellung gezeigt. Der Saugkolben 14 liegt an dem Verschlussstopfen 150 an. Sämtliche Luft aus dem Zylinder 15 ist durch die Stichleitung 68 und das nicht dargestellt Rückschlagventil 8 ausgestoßen worden.

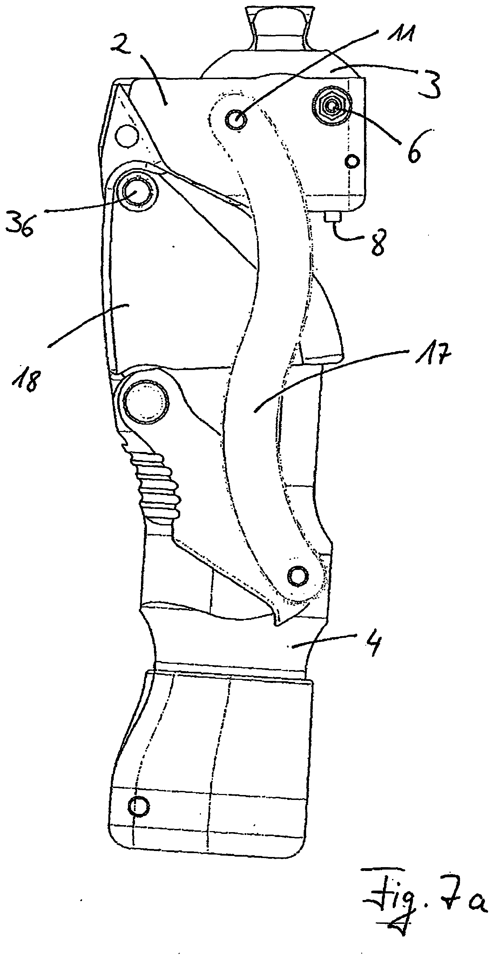

In der Figur 7a ist in der Seitenansicht ein polyzentrisches Prothesenkniegelenk in der Extensionsstellung gezeigt. Es ist der Saugluftanschluss 6, der Auslass 8 sowie die Befestigung des Hebels 17, des so genannten vorderen Lenkers, an der Welle 11 dargestellt, an der wiederum das Zahnrad 12 der Figur 6 festgelegt ist. Der Hebel 17 ist drehfest mit der Welle 11 verbunden und schwenkbar an seinem unteren Ende am Unterteil 4 schwenkbar gelagert. An diesem ist ein zweiter Hebel 18, der so genannte hintere Lenker, gelagert, der über die Achse 36 am Oberteil 2 angeordnet ist. Bei einer Flexion des Oberteils 2 relativ zu dem Unterteil 4 wird die Drehbewegung des Oberteils in eine Drehbewegung des Zahnrades 12 relativ zu dem Oberteil 2 und damit zu der Zahnstange 13 umgewandelt.

Alternativ zu der rotatorischen Festlegung der Welle 11 über die Hebel 17 ist es beispielsweise bei einem monozentrischen Gelenk möglich, eine Kopplung eines feststehenden Zahnrades, das um eine Schwenkachse herum angeordnet ist, über ein Zahnradgetriebe mit der Zahnstange 13 zu verwirklichen. Ebenfalls kann es möglich sein, die Drehbewegung nicht über eine Zahnstange 13 auf einen oszillierenden Saugkolben 14 zur Erzeugung eines Unterdrucks zu übertragen, sondern direkt oder über ein Getriebe auf einen Schwenkkolben, der eine rotatorische Bewegung in dem Oberteil 2 ausführt.

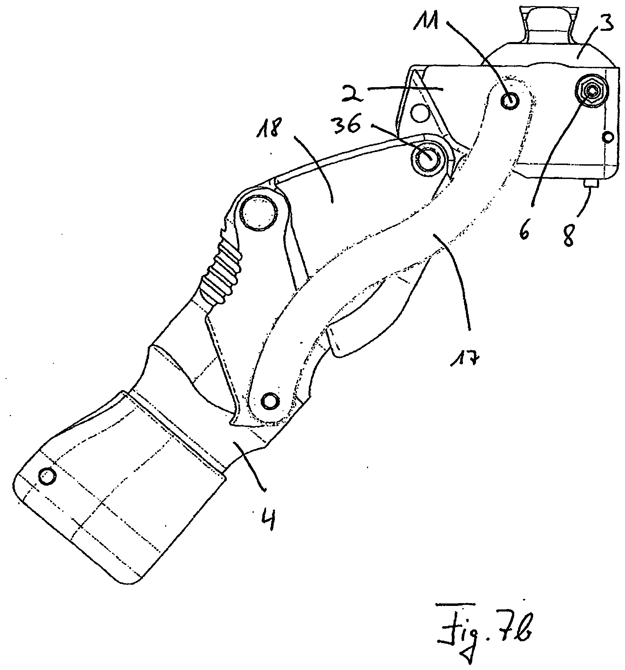

Figur 7b zeigt das polyzentrische Prothesenkniegelenk aus Figur 7a in der Flexionsstellung. Das Unterteil 4 ist gegen das Oberteil 2 relativ verdreht, wobei diese Relativbewegung durch den Hebel 17 über die Welle 11 auf das hinterliegende Zahnrad 12 und damit auf die Zahnstange 13 übertragen wurde. Deutlich zu erkennen ist, dass sich Oberteil 2, Unterteil 4, hinterer Lenker 18 und vorderer Lenker 17 gegeneinander verschoben haben und die Rotationsachse des Kniegelenkes kein eigenständiges Bauteil bildet. Aus den Figuren 7a und 7b ist somit deutlich zu erkennen, dass es sich um ein polyzentrisches Prothesenkniegelenk handelt.

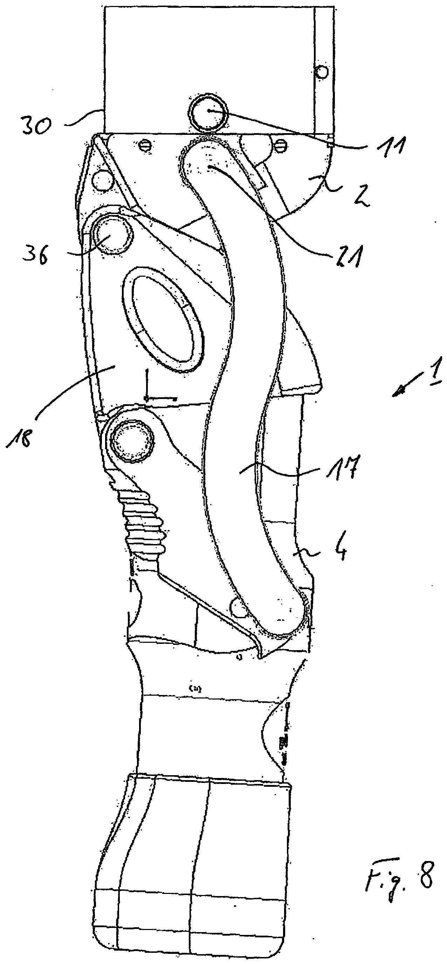

In der Figur 8 ist eine Variante des polyzentrischen Prothesenkniegelenkes 1 der Figur 7 dargestellt. Statt der Integration der Vakuumpumpe 20 in das Oberteil 2 ist vorgesehen, dass ein Aufsatz mit einem Gehäuse 30 auf das Oberteil 2 aufgesetzt wird. In dem Gehäuse 30 ist die Pumpmechanik integriert. Der Antrieb erfolgt dabei ebenfalls durch die Relativbewegung des Oberteils 2 zu dem Unterteil 4. Eine mit dem Hebel 17 verbundene Welle 21 in dem Oberteil 2 ist dabei mit einer Kraftübertragungseinrichtung ausgestattet, die die Relativbewegung zwischen der rotationsstarr an dem Hebel 17 angeordneten Welle und dem Oberteil 2 auf die Antriebswelle 11 der Pumpeneinrichtung in dem Gehäuse 30 überträgt. Alternativ zu der Anordnung der Welle 21 an dem Hebel 17 kann eine Übertragung der Drehbewegung auch durch ein Zahnradgetriebe erfolgen, das in dem Oberteil 2 angeordnet ist.

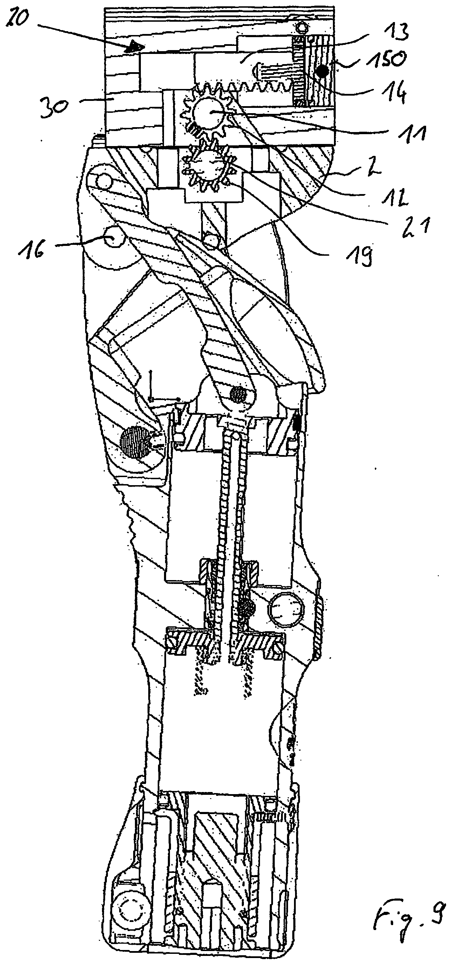

In der Figur 9 ist in einer Schnittdarstellung in Sagittalebene der Aufbau des Prothesenkniegelenkes 1 dargestellt. Die Welle 21 ist mit einem Zahnrad 19 als Kraftübertragungseinrichtung drehfest verbunden. Wird das Oberteil 2 um die Schwenkachse 16 verlagert, dreht sich aufgrund der rotationsstarren Befestigung an dem Hebel 17 das Oberteil 2 relativ zu der Welle 21. Dies führt zu einer Drehbewegung, die auf das Zahnrad 12 der Vakuumpumpe 20 in dem Gehäuse 30 überträgt. Auch hier wird die Drehbewegung von dem Zahnrad 12 über die Zahnstange 13 auf den Saugkolben 14 übertragen. Die oberen Anschlussmittel sind nicht dargestellt und können an dem Gehäuse 20 befestigt werden.

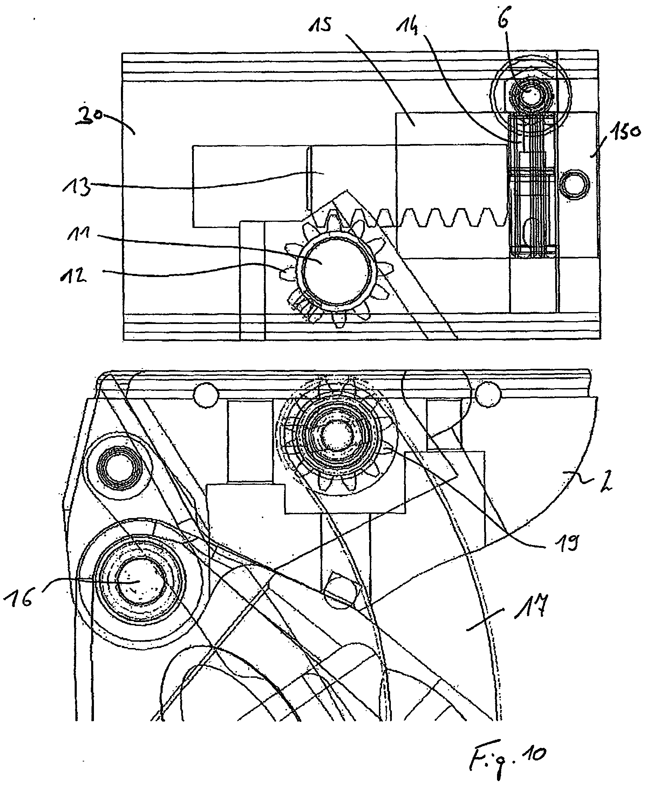

Eine Detailansicht ist in der Figur 10 gezeigt. Es sind die Schwenkachse 16, an der das Oberteil 2 befestigt ist, sowie das Zahnrad 19 und der Hebel 17 zu erkennen. Das Zahnrad 19 steht in dem Oberteil 2 innerhalb einer Nut über eine Grundfläche hervor, so dass das korrespondierende Zahnrad 12 der Pumpe 20 in dem Gehäuse 30 formschlüssig damit in Eingriff treten kann. Das Gehäuse 30 kann auf das Oberteil 2 aufgeschoben und dort verriegelt werden. Auf diese Weise ist es möglich, die Pumpe als ein Modul auszubilden, das nach Bedarf auf das Oberteil 2 aufgesetzt oder von diesem entfernt werden kann. Statt des Gehäuses 30 kann ein oberes Anschlussmittel in Gestalt des Adapters auf die Nut oder die Schiebeführung aufgesetzt und dort verriegelt werden. An der Oberseite des Gehäuses 30 ist eine Befestigungseinrichtung korrespondierend zu der Formgestaltung an der Oberseite des Oberteils 2 ausgebildet, so dass ein Adapter ohne oberes Anschlussmittel auf der Oberseite des Gehäuses 30 befestigt werden kann. In der Figur 10 sind ebenfalls der Zylinder 15, der Saugkolben 14, der Saugluftanschluss 6 sowie der Verschlussstopfen 150 zu erkennen.

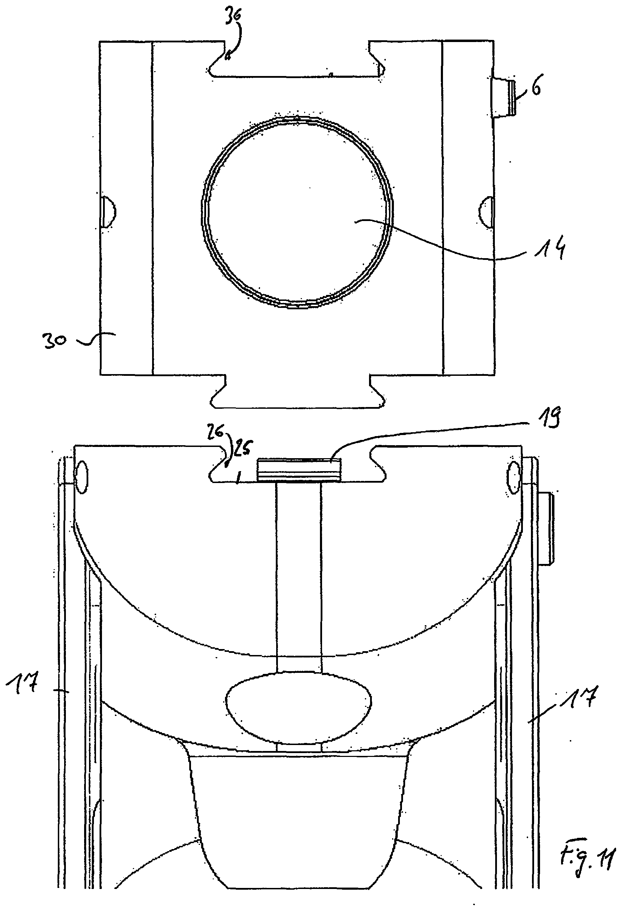

In der Figur 11 ist in der Rückansicht die Ausgestaltung gemäß Figur 10 gezeigt. Es ist das über eine Grundfläche 25 in einer Schwalbenschwanzführung 26 hinausstehende Zahnrad 19 zu erkennen. Ebenfalls ist die korrespondierende Schwalbenschwanzführung an der Oberseite des Gehäuses 30 zu sehen, in der ein Adapter eingeführt werden kann.

## Patentansprüche

1. Prothesenkniegelenk mit einem Oberteil (2), das obere Anschlussmittel (3) aufweist, und einem schwenkbar an dem Oberteil (2) gelagerten Unterteil (4), das untere Anschlussmittel (5) aufweist, **dadurch gekennzeichnet, dass**, um das Prothesenkniegelenk an einem Oberschenkelstumpf festzulegen, eine durch die Relativbewegung des Oberteils (2) zu dem Unterteil (4) angetriebene Vakuumpumpe (20) mit einem Eingang (6) und einem Ausgang (8) dem Prothesenkniegelenk und eine Auslassleitung (8) mit einem Rückschlagventil (9) der Vakuumpumpe (20) zugeordnet ist, wobei die Vakuumpumpe (20) in dem Oberteil (2) integriert ist und mit einem Saugschaft verbunden werden kann.

2. Prothesenkniegelenk nach Anspruch 1, **dadurch gekennzeichnet, dass** die Vakuumpumpe (20) so ausgebildet ist, dass während der Flexion Unterdruck erzeugt wird und während der Extension ein Ausstoß erfolgt.

3. Prothesenkniegelenk nach Anspruch 1 oder 2, **dadurch gekennzeichnet, dass** in der Vakuumpumpe (20) ein Saugkolben (14), insbesondere ein Schwenkkolben oder Linearkolben, angeordnet ist.

4. Prothesenkniegelenk nach Anspruch 3, **dadurch gekennzeichnet, dass** das Prothesenkniegelenk ein monozentrisches oder polyzentrisches Gelenk ist.

5. Prothesenkniegelenk nach einem der voranstehenden Ansprüche, **dadurch gekennzeichnet, dass** eine Kraftübertragungseinrichtung (17) zwischen dem Oberteil (2) und dem Unterteil (4) angeordnet ist, um die Relativbewegung auf die Vakuumpumpe (20) zu übertragen.

6. Prothesenkniegelenk nach Anspruch 5, **dadurch gekennzeichnet, dass** die Kraftübertragungseinrichtung als ein Hebel (17) und/oder ein Zahnrad ausgebildet ist.

7. Prothesenkniegelenk nach einem der voranstehenden Ansprüche, **dadurch gekennzeichnet, dass** ein Rückschlagventil (7) angeordnet ist, das ein Rückströmen von Luft in eine Saugleitung oder einen Saugraum verhindert.

8. Prothesenkniegelenk nach Anspruch 1, **dadurch gekennzeichnet, dass** in oder an der Auslassleitung (8) ein Geräuschdämpfer (10) angeordnet ist.

## Claims

1. Prosthetic knee joint with an upper part (2), which has upper connecting means (3), and with a lower part (4), which is mounted pivotably on the upper part (2) and has lower connecting means (5), **characterized in that**, in order to secure the prosthetic knee joint to a thigh stump, a vacuum pump (20) with an inlet (6) and with an outlet (8) is assigned to the prosthetic knee joint and is driven by the relative movement of the upper part (2) with respect to the lower part (4) and an outlet line (8) with a check valve (9) is assigned to the vacuum pump (20), whereby the vacuum pump (20) is integrated in the upper part (2) and can be connected to a suction socket.

2. Prosthetic knee joint as claimed in claim 1, **characterized in that** the vacuum pump (20) is designed such that underpressure is generated during flexion and an expulsion occurs during extension.

3. Prosthetic knee joint as claimed in claim 1 or 2, **characterized in that** a suction piston (14), in particular an oscillating piston or linear piston, is arranged in the vacuum pump (20).

4. Prosthetic knee joint as claimed in claim 3, **characterized in that** the prosthetic knee joint is a monocentric or polycentric joint.

5. Prosthetic knee joint as claimed in one of the preceding claims, **characterized in that** a force-transferring device (17) is arranged between the upper part (2) and the lower part (4) in order to transfer the relative movement to the vacuum pump (20).

6. Prosthetic knee joint as claimed in claim 5, **characterized in that** the force-transferring device is designed as a lever (17) and/or a toothed wheel.

7. Prosthetic knee joint as claimed in one of the preceding claims, **characterized in that** a check valve (7) is arranged to prevent a backflow of air into a suction line or a suction space.

8. Prosthetic knee joint as claimed in claim 1, **characterized in that** a noise damper (10) is arranged in or on the outlet line (8).

## Revendications

1. Prothèse de l'articulation du genou avec une partie supérieure (2), qui comprend un moyen de raccordement supérieur (3), et une partie inférieure (4) montée avec possibilité de pivotement sur la partie supérieure (2), qui comprend un moyen de raccordement inférieur (5), **caractérisée en ce que**, pour immobiliser la prothèse de l'articulation du genou sur un moignon de la cuisse, une pompe à vide (20) entraînée par le mouvement relatif de la partie supérieure (2) par rapport à la partie inférieure (4) et comprenant une entrée (6) et une sortie (8), et associée à la prothèse de l'articulation du genou, et un conduit de sortie (8) comprenant un clapet antiretour (9) est associé à la pompe à vide, dans laquelle la pompe à vide (20) est intégrée dans la partie supérieure, et peut être reliée à une emboîture à dépression.

2. Prothèse de l'articulation du genou selon la revendication 1, **caractérisée en ce que** la pompe à vide (20) est ainsi réalisée que, pendant la flexion, elle engendre une dépression et qu'un échappement a lieu pendant l'extension.

3. Prothèse de l'articulation du genou selon la revendication 1 ou 2, **caractérisée en ce qu'**un piston d'aspiration (14), en particulier un piston pivotant ou un piston linéaire, est agencé dans la pompe à vide (20).

4. Prothèse de l'articulation du genou selon la revendication 3, **caractérisée en ce que** la prothèse de l'articulation du genou est une articulation monocentrique ou polycentrique.

5. Prothèse de l'articulation du genou selon l'une des revendications précédentes, **caractérisée en ce qu'**un système de transmission de force (17) est agencé entre la partie supérieure (2) et la partie inférieure (4), afin de transmettre le mouvement relatif à la pompe à vide (20).

6. Prothèse de l'articulation du genou selon la revendication 5, **caractérisée en ce que** le système de transmission de force est réalisé sous forme de levier (17) ou sous forme de roue dentée.

7. Prothèse de l'articulation du genou selon l'une des revendications précédentes, **caractérisée en ce qu'**il est prévu un clapet antiretour (7), qui empêche un reflux d'air vers une conduit d'aspiration ou vers une chambre d'aspiration.

8. Prothèse de l'articulation du genou selon la revendication 1, **caractérisée en ce qu'**un silencieux (10) est agencé dans ou sur la conduite d'échappement (8).
